# EUROPÄISCHE PATENTSCHRIFT

(11) **EP 1 259 623 B1**
(45) Veröffentlichungstag und Bekanntmachung des Hinweises auf die Patenterteilung: **29.04.2009**
(21) Anmeldenummer: 01929378.6
(22) Anmeldetag: 01.03.2001
(51) Int. Cl.: C12N 15/54, C12N 15/82, C12N 9/10, C12Q 1/48

(54) **ASPARTAT-CARBAMYLTRANSFERASE ALS HERBIZIDES TARGET**
ASPARTATE CARBAMYLTRANSFERASE AS HERBICIDAL TARGET
ASPARTATE-CARBAMYLE TRANSFERASE UTILISEE COMME CIBLE POUR HERBICIDES

(30) Priorität: 02.03.2000 DE 10009937
(43) Veröffentlichungstag der Anmeldung: 27.11.2002
(73) Patentinhaber: BASF SE, 67056 Ludwigshafen (DE)
(72) Erfinder: EHRHARDT, Thomas, 67346 Speyer (DE); LERCHL, Jens, 26831 Svalöv (SE); STITT NIGEL, Marc, 14469 Potsdam (DE); ZRENNER, Rita, 14476 Golm (DE); RITTER, Tina, Maria, 69151 Dilsberg (DE)
(74) Vertreter: Fitzner, Ulrich
(86) Internationale Anmeldenummer: PCT/EP2001/002284
(87) Internationale Veröffentlichungsnummer: WO 2001/064900

(56) Entgegenhaltungen:
- OVERDUIN BERT ET AL: "The Asc locus for resistance to Alternaria stem canker in tomato does not encode the enzyme aspartate carbamoyltransferase." MOLECULAR & GENERAL GENETICS, Bd. 240, Nr. 1, 1993, Seiten 43-48, XP002175083 ISSN: 0026-8925
- WILLIAMSON CYNTHIA L ET AL: "Molecular cloning and characterization of the pyrB1 and pyrB2 genes encoding aspartate transcarbamoylase in pea (Pisum sativum L.)." PLANT PHYSIOLOGY (ROCKVILLE), Bd. 105, Nr. 1, 1994, Seiten 377-384, XP002175084 ISSN: 0032-0889 in der Anmeldung erwähnt -& DATABASE EMPLN [Online] EMBL Heidelberg; AC M96981, ID PSPYRB1A, 2. Juni 1994 (1994-06-02) WILLIANSON CL AND SLOCUM R D: XP002175085 -& DATABASE EMPLN [Online] EMBL Heidelberg; AC L15798 ID PSPYRB2A, 2. Juni 1994 (1994-06-02) WILLIAMSON C L AND SLOCUM R D: XP002175086
- DATABASE BIOSIS [Online] BIOSCIENCES INFORMATION SERVICE, PHILADELPHIA, PA, US; 1989 ACASTER M A ET AL: "CARBAMOYLTRANSFERASE REACTIONS IN PLANTS A SURVEY FOR ENZYMIC DIVERSITY AND THE POTENTIAL FOR HERBICIDAL ACTIVITY OF TRANSITION STATE ANALOGUE INHIBITORS" Database accession no. PREV199089052659 XP002175087 & JOURNAL OF EXPERIMENTAL BOTANY, Bd. 40, Nr. 219, 1989, Seiten 1121-1126, ISSN: 0022-0957
- DATABASE BIOSIS [Online] BIOSCIENCES INFORMATION SERVICE, PHILADELPHIA, PA, US; 1984 LOVATT C J ET AL: "ASPARTATE CARBAMYL TRANSFERASE EC-2.1.3.2 SITE OF END PRODUCT INHIBITION OF THE OROTATE PATHWAY IN INTACT CELLS OF CUCURBITA-PEPO CULTIVAR EARLY-PROLIFIC-STRAIGHTNECK" Database accession no. PREV198478087023 XP002175088 in der Anmeldung erwähnt & PLANT PHYSIOLOGY (BETHESDA), Bd. 75, Nr. 3, 1984, Seiten 511-515, ISSN: 0032-0889
- HOFGEN RANIER ET AL: "Repression of Acetolactate Synthase Activity through Antisense Inhibition. Molecular and Biochemical Analysis of Transgenic Potato (Solanum tuberosum L. cv Desiree) Plants" PLANT PHYSIOLOGY, AMERICAN SOCIETY OF PLANT PHYSIOLOGISTS, ROCKVILLE, MD, US, Bd. 107, Nr. 2, 1995, Seiten 469-477, XP002145367 ISSN: 0032-0889 in der Anmeldung erwähnt

### SEQUENCE LISTING

<110> BASF Aktiengesellschaft
<120> Aspartat-Carbamyltransferase als herbizides Target
<130> BASF-OZ-0050-51219-PCT
<140>
   <141>
<160> 6
<170> PatentIn Ver. 2.1
<210> 1
   <211> 1344
   <212> DNA
   <213> Solanum tuberosum
<220>
   <221> CDS
   <222> (106)..(1236)
<400> 1
   ctcgagcagc tccaatctcc gacagaacca aacgtcatcg gaaaactctt gacgatctcc 60
   gccacactgc cgccgttgtt cctctctccg gtgagtgatt tcaat atg act att tcc 117
                                                     Met Thr Ile Ser
                                                      1
   gca aca ttt tct tcc cat gga aaa ata ctc atg tca cct ctt aga aag   165
   Ala Thr Phe Ser Ser His Gly Lys Ile Leu Met Ser Pro Leu Arg Lys
    5                  10                  15                  20
   agt gaa tgg gca aat caa cct gtg ctc tgt aaa tct gtt gaa ttg ttt   213
   Ser Glu Trp Ala Asn Gln Pro Val Leu Cys Lys Ser Val Glu Leu Phe
                   25                  30                  35
   aaa aat gga cga aat caa tat cgt atg tca aca aat agc aac aaa ttc   261
   Lys Asn Gly Arg Asn Gln Tyr Arg Met Ser Thr Asn Ser Asn Lys Phe
               40                  45                  50
   caa tgc cga gca ctg gag atc gaa aat aaa tct tca act aag ttt cac   309
   Gln Cys Arg Ala Leu Glu Ile Glu Asn Lys Ser Ser Thr Lys Phe His
           55                  60                  65
   ctt gat gat gta att gaa tct caa caa ttt gat aga gaa act ctt agt   357
   Leu Asp Asp Val Ile Glu Ser Gln Gln Phe Asp Arg Glu Thr Leu Ser
       70                  75                  80
   gcg ata ttt gaa gta gca cga gag atg gag aaa atc gag aag aat tcg   405
   Ala Ile Phe Glu Val Ala Arg Glu Met Glu Lys Ile Glu Lys Asn Ser
    85                  90                  95                 100
   att gga gga aga agc gag att ctt aag ggc tat ctt atg gct act ctg   453
   Ile Gly Gly Arg Ser Glu Ile Leu Lys Gly Tyr Leu Met Ala Thr Leu
                   105                 110                 115
   ttt tat gaa cct tca act aga act agg ctt tca ttt gaa tct gct atg   501
   Phe Tyr Glu Pro Ser Thr Arg Thr Arg Leu Ser Phe Glu Ser Ala Met
               120                 125                 130
   aag cgt tta gga gga gaa gta tta aca acc gaa aat gct cgt gaa ttt    549
   Lys Arg Leu Gly Gly Glu Val Leu Thr Thr Glu Asn Ala Arg Glu Phe
           135                 140                 145
   tca tct gca gca aag ggg gaa aca cta gaa gat aca att aga act gtt    597
   Ser Ser Ala Ala Lys Gly Glu Thr Leu Glu Asp Thr Ile Arg Thr Val
       150                 155                 160
   gaa ggt tac tct gat atc att gtt atg agg cat ttc gaa agt ggt gct    645
   Glu Gly Tyr Ser Asp Ile Ile Val Met Arg His Phe Glu Ser Gly Ala
   165                 170                 175                 180
   gct aga aga gcg gcg aca act gca tct att ccg att ata aat gca gga    693
   Ala Arg Arg Ala Ala Thr Thr Ala Ser Ile Pro Ile Ile Asn Ala Gly
                   185                 190                 195
   gat ggt ccg gga caa cac ccg act cag gca ctt cta gat gtg tat aca    741
   Asp Gly Pro Gly Gln His Pro Thr Gln Ala Leu Leu Asp Val Tyr Thr
               200                 205                 210
   att gaa cga gaa ata ggg aaa ctc gat ggt ata aac gtt gct ctt gtt    789
   Ile Glu Arg Glu Ile Gly Lys Leu Asp Gly Ile Asn Val Ala Leu Val
           215                 220                 225
   ggt gat cta gca tac ggg agg aca gtt cgt tca ctt gct cat ttg ctt    837
   Gly Asp Leu Ala Tyr Gly Arg Thr Val Arg Ser Leu Ala His Leu Leu
       230                 235                 240
   gca atg tat gaa gat gtg aaa att tac ttt gta tcc cct gat gtt gtt    885
   Ala Met Tyr Glu Asp Val Lys Ile Tyr Phe Val Ser Pro Asp Val Val
   245                 250                 255                 260
   aaa atg aag gat gac ata aag gat tac ttg aca tca atg ggg gtt caa    933
   Lys Met Lys Asp Asp Ile Lys Asp Tyr Leu Thr Ser Met Gly Val Gln
                   265                 270                 275
   tgg gaa gaa aat gct gat ttg atc gag gtg gct tct aaa tgt gac gtg    981
   Trp Glu Glu Asn Ala Asp Leu Ile Glu Val Ala Ser Lys Cys Asp Val
               280                 285                 290
   gtg tat caa act cgt att caa aga gag aga ttt gga gag agg gtt gat    1029
   Val Tyr Gln Thr Arg Ile Gln Arg Glu Arg Phe Gly Glu Arg Val Asp
           295                 300                 305
   ttg tat gaa gaa gct cga ggg aag tat atc gtt gat atg agt gtc gta    1077
   Leu Tyr Glu Glu Ala Arg Gly Lys Tyr Ile Val Asp Met Ser Val Val
       310                 315                 320
   aat gct atg cag aaa cat gct gta gtg atg cat cct ttg cca aga ctt    1125
   Asn Ala Met Gln Lys His Ala Val Val Met His Pro Leu Pro Arg Leu
   325                 330                 335                 340
   gat gag att act gtt gat gtt gat ggt gat ccg agg gct gct tat ttc    1173
   Asp Glu Ile Thr Val Asp Val Asp Gly Asp Pro Arg Ala Ala Tyr Phe
                   345                 350                 355
   aga caa gct aag aat ggt ctt tac att cgg atg gcg ctt ttg aag ctt    1221
   Arg Gln Ala Lys Asn Gly Leu Tyr Ile Arg Met Ala Leu Leu Lys Leu
               360                 365                 370
   cta ctc ctt ggt tgg tgaaagatgc aaacttttta ttttgcgaaa tcgtgcttct   1276
   Leu Leu Leu Gly Trp
           375
   tgtctggatt ctgctgttgt tgaattcaag agttttgtta acttgtttta gcataaaaaa 1336
   aaaaaaaa                                                          1344
<210> 2
   <211> 377
   <212> PRT
   <213> Solanum tuberosum
<400> 2
   Met Thr Ile Ser Ala Thr Phe Ser Ser His Gly Lys Ile Leu Met Ser
    1               5                  10                  15
   Pro Leu Arg Lys Ser Glu Trp Ala Asn Gln Pro Val Leu Cys Lys Ser
               20                  25                  30
   Val Glu Leu Phe Lys Asn Gly Arg Asn Gln Tyr Arg Met Ser Thr Asn
           35                  40                  45
   Ser Asn Lys Phe Gln Cys Arg Ala Leu Glu Ile Glu Asn Lys Ser Ser
       50                  55                  60
   Thr Lys Phe His Leu Asp Asp Val Ile Glu Ser Gln Gln Phe Asp Arg
   65                  70                  75                      80
   Glu Thr Leu Ser Ala Ile Phe Glu Val Ala Arg Glu Met Glu Lys Ile
                   85                  90                  95
   Glu Lys Asn Ser Ile Gly Gly Arg Ser Glu Ile Leu Lys Gly Tyr Leu
               100                 105                 110
   Met Ala Thr Leu Phe Tyr Glu Pro Ser Thr Arg Thr Arg Leu Ser Phe
           115                 120                 125
   Glu Ser Ala Met Lys Arg Leu Gly Gly Glu Val Leu Thr Thr Glu Asn
       130                 135                 140
   Ala Arg Glu Phe Ser Ser Ala Ala Lys Gly Glu Thr Leu Glu Asp Thr
   145                 150                 155                 160
   Ile Arg Thr Val Glu Gly Tyr Ser Asp Ile Ile Val Met Arg His Phe
                   165                 170                 175
   Glu Ser Gly Ala Ala Arg Arg Ala Ala Thr Thr Ala Ser Ile Pro Ile
               180                 185                 190
   Ile Asn Ala Gly Asp Gly Pro Gly Gln His Pro Thr Gln Ala Leu Leu
           195                 200                 205
   Asp Val Tyr Thr Ile Glu Arg Glu Ile Gly Lys Leu Asp Gly Ile Asn
       210                 215                 220
   Val Ala Leu Val Gly Asp Leu Ala Tyr Gly Arg Thr Val Arg Ser Leu
   225                 230                 235                 240
   Ala His Leu Leu Ala Met Tyr Glu Asp Val Lys Ile Tyr Phe Val Ser
                   245                 250                 255
   Pro Asp Val Val Lys Met Lys Asp Asp Ile Lys Asp Tyr Leu Thr Ser
               260                 265                 270
   Met Gly Val Gln Trp Glu Glu Asn Ala Asp Leu Ile Glu Val Ala Ser
           275                 280                 285
   Lys Cys Asp Val Val Tyr Gln Thr Arg Ile Gln Arg Glu Arg Phe Gly
       290                 295                 300
   Glu Arg Val Asp Leu Tyr Glu Glu Ala Arg Gly Lys Tyr Ile Val Asp
   305                 310                 315                 320
   Met Ser Val Val Asn Ala Met Gln Lys His Ala Val Val Met His Pro
                   325                 330                 335
   Leu Pro Arg Leu Asp Glu Ile Thr Val Asp Val Asp Gly Asp Pro Arg
               340                 345                 350
   Ala Ala Tyr Phe Arg Gln Ala Lys Asn Gly Leu Tyr Ile Arg Met Ala
           355                 360                 365
   Leu Leu Lys Leu Leu Leu Leu Gly Trp
       370                 375
<210> 3
   <211> 617
   <212> DNA
   <213> Physcomitrella patens
<220>
   <221> CDS
   <222> (1)..(354)
<400> 3
   ggc acg agg aag gat gat atc aaa gat cat tta acc gag tca gga gtg    48
   Gly Thr Arg Lys Asp Asp Ile Lys Asp His Leu Thr Glu Ser Gly Val
     1               5                  10                  15
   gat tgg gaa gaa tcc tcc gat ttg atg caa gtg gca tca aag tgt gat    96
   Asp Trp Glu Glu Ser Ser Asp Leu Met Gln Val Ala Ser Lys Cys Asp
                20                  25                  30
   gta ata tac cag act cgc atc caa caa gag agg ttc ggt gat cgc atc   144
   Val Ile Tyr Gln Thr Arg Ile Gln Gln Glu Arg Phe Gly Asp Arg Ile
            35                  40                  45
   gac ctc tac aat gag gct cga ggg aag tac ata gtg gat acc aaa gtc   192
   Asp Leu Tyr Asn Glu Ala Arg Gly Lys Tyr Ile Val Asp Thr Lys Val
        50                  55                  60
   atg gat gtg ttg ccc aag cac gga gtg gtc ctc cat ccg cta cca cgg   240
   Met Asp Val Leu Pro Lys His Gly Val Val Leu His Pro Leu Pro Arg
    65                  70                  75                  80
   ctt gat gaa ata act gtg gag gtg gac agt gat ccc cga gct gct tat   288
   Leu Asp Glu Ile Thr Val Glu Val Asp Ser Asp Pro Arg Ala Ala Tyr
                    85                  90                  95
   ttc cgg caa gct aag aac ggt ctt tac att cgg atg gcc ctc ttg aag   336
   Phe Arg Gln Ala Lys Asn Gly Leu Tyr Ile Arg Met Ala Leu Leu Lys
               100                 105                 110
   ttg ctt ctt ctc ggc tac taaggcaggt caactgtcac agtgtttgtt          384
   Leu Leu Leu Leu Gly Tyr
           115
   cttcgggaag tgagtgctgt gattgagata ttggattgag agtagctcag ccctctttcg 444
   tgagtagtga tgatgaaatt caactgaaat tcaactaggc aagcagtaac agaaagacgt 504
   agatgcaaga atttgtaaat gacaatttgg ctgtcgagtt ctgaccctca gagccacttt 564
   aattgtacat acgcagttgc catagcattt aatgctaaaa aaaaaaaaaa aaa        617
<210> 4
   <211> 118
   <212> PRT
   <213> Physcomitrella patens
<400> 4
   Gly Thr Arg Lys Asp Asp Ile Lys Asp His Leu Thr Glu Ser Gly Val
   1               5                   10                  15
   Asp Trp Glu Glu Ser Ser Asp Leu Met Gln Val Ala Ser Lys Cys Asp
               20                  25                  30
   Val Ile Tyr Gln Thr Arg Ile Gln Gln Glu Arg Phe Gly Asp Arg Ile
           35                  40                  45
   Asp Leu Tyr Asn Glu Ala Arg Gly Lys Tyr Ile Val Asp Thr Lys Val
       50                  55                  60
   Met Asp Val Leu Pro Lys His Gly Val Val Leu His Pro Leu Pro Arg
   65                  70                  75                  80
   Leu Asp Glu Ile Thr Val Glu Val Asp Ser Asp Pro Arg Ala Ala Tyr
                   85                  90                  95
   Phe Arg Gln Ala Lys Asn Gly Leu Tyr Ile Arg Met Ala Leu Leu Lys
               100                 105                 110
   Leu Leu Leu Leu Gly Tyr
           115
<210> 5
   <211> 1251
   <212> DNA
   <213> Physcomitrella patens
<220>
   <221> CDS
   <222> (1) .. (933)
   <223> c_pp015013185r
<400> 5
   agg ttt cca tta cag gat gta ctg gaa gcg cag cag ttc gac agg aaa    48
   Arg Phe Pro Leu Gln Asp Val Leu Glu Ala Gln Gln Phe Asp Arg Lys
    1               5                  10                  15
   ctt ctg aac tct atc ttt gag gtg gca agg gaa atg gaa gac ata gag    96
   Leu Leu Asn Ser Ile Phe Glu Val Ala Arg Glu Met Glu Asp Ile Glu
               20                  25                  30
   agg gat gct gct gga tcg gag ctg ctg aag gga ttc tta atg gct acc   144
   Arg Asp Ala Ala Gly Ser Glu Leu Leu Lys Gly Phe Leu Met Ala Thr
           35                  40                  45
   ctg ttt tac gaa cct tcc act cgt act agg ctt tcg ttc gaa tcc gct   192
   Leu Phe Tyr Glu Pro Ser Thr Arg Thr Arg Leu Ser Phe Glu Ser Ala
       50                  55                  60
   atg aag agg ctg ggt gga gaa gtt ttg acg act gaa aat gct cgt gaa   240
   Met Lys Arg Leu Gly Gly Glu Val Leu Thr Thr Glu Asn Ala Arg Glu
   65                  70                  75                  80
   ttt tct tca gct gcc aag ggc gag acc ctg gaa gat acc att aga aca   288
   Phe Ser Ser Ala Ala Lys Gly Glu Thr Leu Glu Asp Thr Ile Arg Thr
                   85                  90                  95
   gtc gag ggc tat tcg gat att att gtg ctc agg cac ttc gag agt ggg   336
   Val Glu Gly Tyr Ser Asp Ile Ile Val Leu Arg His Phe Glu Ser Gly
               100                 105                 110
   gca gcc aag aag gct gcc aat aca gcg aac att cct atc ata aat gca   384
   Ala Ala Lys Lys Ala Ala Asn Thr Ala Asn Ile Pro Ile Ile Asn Ala
           115                 120                 125
   gga gat ggt cct ggt cag cat cct aca cag gcg ctg ctc gac atg tat   432
   Gly Asp Gly Pro Gly Gln His Pro Thr Gln Ala Leu Leu Asp Met Tyr
       130                 135                 140
   aca ata aaa cga gag att ggc cat ctg gat aac ata aga gtg ggg ttg   480
   Thr Ile Lys Arg Glu Ile Gly His Leu Asp Asn Ile Arg Val Gly Leu
   145                 150                 155                 160
   gtg ggt gat tta gct aat ggg cga act gta cga tca ttg gct tat ctg   528
   Val Gly Asp Leu Ala Asn Gly Arg Thr Val Arg Ser Leu Ala Tyr Leu
                   165                 170                 175
   ctt gcg aag tac gat gga gtc aaa att tac ttt gtt gct cct gat gtg   576
   Leu Ala Lys Tyr Asp Gly Val Lys Ile Tyr Phe Val Ala Pro Asp Val
               180                 185                 190
   gtc aag atg aag gat gat atc aag gac cac ctg act gat tca gga gtc   624
   Val Lys Met Lys Asp Asp Ile Lys Asp His Leu Thr Asp Ser Gly Val
           195                 200                 205
   gat tgg gaa gaa tcc act gac ctg atg gaa gtg gca tca aag tgt gat   672
   Asp Trp Glu Glu Ser Thr Asp Leu Met Glu Val Ala Ser Lys Cys Asp
       210                  215                  220
   gtg ata tac caa act cgt atc caa cgt gag agg ttt ggt gac cgc att    720
   Val Ile Tyr Gln Thr Arg Ile Gln Arg Glu Arg Phe Gly Asp Arg Ile
   225                 230                 235                 240
   gac ttg tac aat gcg gct cgt gga aag tat att gtg gat acc aag gtt    768
   Asp Leu Tyr Asn Ala Ala Arg Gly Lys Tyr Ile Val Asp Thr Lys Val
                   245                 250                 255
   atg aat gtg tta ccc aag cat gcg gtg gtt ctc cat cct cta cca cgt    816
   Met Asn Val Leu Pro Lys His Ala Val Val Leu His Pro Leu Pro Arg
               260                 265                 270
   ctc gat gag ata act gtg gaa gtg gac agt gat ccc cga gct gct tac    864
   Leu Asp Glu Ile Thr Val Glu Val Asp Ser Asp Pro Arg Ala Ala Tyr
           275                 280                 285
   ttc cgt caa gct aag aat ggt ctg cac att cgg atg gcc tta ctc aag    912
   Phe Arg Gln Ala Lys Asn Gly Leu His Ile Arg Met Ala Leu Leu Lys
       290                 295                 300
   ctg ctt ctt ctt ggc tac taa gggagaagct catgtagcaa ttttggtgca       963
   Leu Leu Leu Leu Gly Tyr
   305                 310
   caggattagg ctgctgagtg ttgcgcatag gattgagagt agtgaacagg gtttcgtgag 1023
   gaggtcacat tgaatataaa ttcaatcgga gaaggatttt ctgtaaactg acaaaagcaa 1083
   cctagtgcaa aagttcctac gaataatttt tttgttatgt tctgactcca gaactttctg 1143
   aaattgtaca tagagagctg cctttcattc aatgtcgcaa acaactgggc acctcctaga 1203
   aattggcggc tttgctaaaa aaaaaaaaaa aaaaaaaaaa aaaaaaaa               1251
<210> 6
   <211> 310
   <212> PRT
   <213> Physcomitrella patens
<400> 6
   Arg Phe Pro Leu Gln Asp Val Leu Glu Ala Gln Gln Phe Asp Arg Lys
    1               5                  10                  15
   Leu Leu Asn Ser Ile Phe Glu Val Ala Arg Glu Met Glu Asp Ile Glu
               20                  25                  30
   Arg Asp Ala Ala Gly Ser Glu Leu Leu Lys Gly Phe Leu Met Ala Thr
           35                  40                  45
   Leu Phe Tyr Glu Pro Ser Thr Arg Thr Arg Leu Ser Phe Glu Ser Ala
       50                  55                  60
   Met Lys Arg Leu Gly Gly Glu Val Leu Thr Thr Glu Asn Ala Arg Glu
   65                  70                  75                  80
   Phe Ser Ser Ala Ala Lys Gly Glu Thr Leu Glu Asp Thr Ile Arg Thr
                   85                  90                  95
   Val Glu Gly Tyr Ser Asp Ile Ile Val Leu Arg His Phe Glu Ser Gly
               100                 105                 110
   Ala Ala Lys Lys Ala Ala Asn Thr Ala Asn Ile Pro Ile Ile Asn Ala
           115                 120                 125
   Gly Asp Gly Pro Gly Gln His Pro Thr Gln Ala Leu Leu Asp Met Tyr
       130                 135                 140
   Thr Ile Lys Arg Glu Ile Gly His Leu Asp Asn Ile Arg Val Gly Leu
   145                 150                 155                 160
   Val Gly Asp Leu Ala Asn Gly Arg Thr Val Arg Ser Leu Ala Tyr Leu
                   165                 170                 175
   Leu Ala Lys Tyr Asp Gly Val Lys Ile Tyr Phe Val Ala Pro Asp Val
               180                 185                 190
   Val Lys Met Lys Asp Asp Ile Lys Asp His Leu Thr Asp Ser Gly Val
           195                 200                 205
   Asp Trp Glu Glu Ser Thr Asp Leu Met Glu Val Ala Ser Lys Cys Asp
       210                 215                 220
   Val Ile Tyr Gln Thr Arg Ile Gln Arg Glu Arg Phe Gly Asp Arg Ile
   225                 230                 235                 240
   Asp Leu Tyr Asn Ala Ala Arg Gly Lys Tyr Ile Val Asp Thr Lys Val
                   245                 250                 255
   Met Asn Val Leu Pro Lys His Ala Val Val Leu His Pro Leu Pro Arg
               260                 265                 270
   Leu Asp Glu Ile Thr Val Glu Val Asp Ser Asp Pro Arg Ala Ala Tyr
           275                 280                 285
   Phe Arg Gln Ala Lys Asn Gly Leu His Ile Arg Met Ala Leu Leu Lys
       290                 295                 300
   Leu Leu Leu Leu Gly Tyr
   305                 310

## Beschreibung

Die vorliegende Erfindung betrifft die Identifizierung pflanzlicher Aspartat-Carbamyltransferase (E.C. 2.1.3.2) als neues Ziel für herbizide Wirkstoffe. Die vorliegende Erfindung betrifft weiterhin die Verwendung der DNA-Sequenz SEQ-ID No. 1 oder SEQ-ID No. 3 oder SEQ-ID No. 5 oder Teile oder Derivate kodierend für ein pflanzliches Polypeptid mit Aspartat-Carbamyltransferase Aktivität zur Herstellung eines Testsystems zur Identifizierung von Inhibitoren der pflanzlichen Aspartat-Carbamyltransferase. Die Erfindung betrifft auch ein Verfahren bzw. ein Testsystem zum Auffinden von Substanzen, die die Aktivität der pflanzlichen Aspartat-Carbamyltransferase inhibieren.

Pflanzen sind in der Lage, aus Kohlendioxid, Wasser und anorganischen Salzen ihre Zellkomponenten zu synthetisieren.

Dieser Prozeß ist nur möglich, indem biochemische Reaktionen zum Aufbau organischer Substanzen genutzt werden. Nukleotide werden in Pflanzen de novo synthetisiert. Als Bestandteil der Nukleinsäuren kommt ihnen besondere Bedeutung zu. In kovalenter Bindung aktivieren Nukleotide Kohlenhydrate für die Biosynthese von Polysacchariden. Ferner aktivieren sie Kopfgruppen für die Biosynthese von Lipiden. Nukleotide sind in nahezu alle Stoffwechselwege eingebunden. Nukleosidtriphosphate, vor allem ATP, treiben die meisten energieaufwändigen Reaktionen der Zelle. Adeninnukleotide sind darüber hinaus auch als Komponente in essentiellen Faktoren wie Coenzym A, sowie Nicotinamid- und Flavin-Coenzymen zu finden, die an vielen zellulären Reaktionen beteiligt sind. Die gekoppelte Hydrolyse von Guanosin-5'-triphosphat (GTP) definiert für diverse zelluläre Prozesse, wie Proteintranslation, Microtubuli-Assemblierung, vesikulären Transport, Signaltransduktion und Zellteilung eine Reaktionsrichtung. Ferner stellen Nukleotide die Ausgangsmetabolite zur Biosynthese von Methylxanthinen wie Coffein und Theobromin in Pflanzenfamilien der Rubiaceae und Theaceae dar.

Da Pflanzen auf einen effektiven Nukleotidstoffwechsel angewiesen sind, läßt sich vermuten, daß sich Enzyme, die an der Nucleotidbiosynthese beteiligt sind, als Zielprotein (Target) für Herbizide eignen. So wurden bereits Wirkstoffe beschrieben, welche die pflanzliche de novo Purinbiosynthese inhibieren. Beispielhaft ist der Naturstoff Hydanthocidin zu nennen, welcher nach Phosphorylierung in planta die Adenylosuccinat-Synthetase (ASS), inhibiert (Siehl et al., Plant Physiol. 110(1996), 753-758).

Sowohl die Enzymreaktionen der de novo Purinbiosynthese als auch die Enzymreaktionen der de novo Pyrimidinbiosynthese sind zur Regulation des Nukleotidstoffwechsels wichtig. Eines dieser Enzyme ist die Aspartat-Carbamoyltransferase (ATCase). Die ATCase katalysiert im zweiten Schritt der Pyrimidinbiosynthese die Verknüpfung von Carbamoylphosphat und Aspartat zu Carbamoylaspartat, dem Substrat der Dihydroorotase:

Carbamoylphosphat ist auch das Substrat der Ornithin Carbamoyltransferase in der Arginin-Biosynthese. In Pflanzen und vielen weiteren Eukaryonten und Prokaryonten wird die ATCase allosterisch durch Pyrimidinnukleotide feedback reguliert. Auf die ATC-ase aus Zucchini wirken UMP und UDP stärker inhibierend als CMP und CDP (Lovatt und Cheng, Plant Physiol. 75(1984), 511-515). In *Saccharomyces cerevisiae* codiert das URA2 Gen ein Fusionsprotein mit ATCase- und Carbamoylphosphat Synthetase-Aktivität (CPS). In höheren Eukaryonten ist die ATCase Bestandteil eines trifunktionellen Proteins (CAD), welches neben ATCase und CPS auch die Dihydroorotase beinhaltet. In *E. coli* liegt die ATCase als heteromerer Komplex dreier katalytischer und dreier regulatorischer Untereinheiten vor. Für die ATCase aus Pflanzen wurde eine regulatorische Untereinheit nicht gefunden.

Pflanzliche ATCase Gene wurden zunächst über funktionelle Komplementation von E. coli (Williamson und Slocum, Plant Physiol. 105(1994), 377-384) und Saccharomyces cerevisiae pyrB-Mutanten (Nasr et al., Mol. Gen. Genet. 244(1994), 23-32) isoliert. Inzwischen finden sich ATCase Gene aus verschiedenen Pflanzenspezies, wie *Arabidopsis thaliana* (AB024036), *Pisum sativum* (M96981, U05293, L15798), *Lycopersicon esculentum* (X74072), *Oryza sativa* (C27734) und *Glycine max* (AW132309) in den Datenbanken. Aus Erbse wurden drei ATCase Isoformen isoliert. Mindestens eine der Isoformen läßt sich in *E.coli* funktionell exprimieren (Williamson und Slocum, Plant Physiol. 105(1994), 377-384).

Die ATCase-Aktivität wurde in Pflanzen vornehmlich in Chloroplasten detektiert (Shibata et al., Plant Physiol. 80(1986), 126-129). Das Substratanalogon N-(phosphonacetyl)-L-aspartat (PALA) ist ein potenter Inhibitor der ATCase aus Pro- und Eukaryonten und wirkt antimikrobiell. Ferner zeigen Acaster et al., (1989, Journal of Experimental Botany 40 (219), 1121-1126) dass PALA und PALO (N-(phosphonacetyl)-L-ornithine) starke in-vitro Inhibitoren von Aspartat Carbamoyltransferasen aus neun Spezies sind, die herbizide Wirkung hingegen schwach ist.

Der Nachweis der Eignung eines Enzyms als Target für Herbizide kann durch Verringerung der Enzymaktivität zum Beispiel mittels Antisensetechnik in transgenen Pflanzen erfolgen. Wird durch Einbringen einer Antisense-DNA für ein bestimmtes Gen in eine Pflanze ein verringertes Wachstum bewirkt, so deutet dies auf die Eignung des in seiner Aktivität reduzierten Enzyms als Wirkort für herbizide Wirkstoffe hin. Beispielsweise führt die Antisense-Inhibierung der Acetolactat-Synthase (ALS) in transgenen Kartoffelpflanzen zu vergleichbaren Phänotypen, wie die Behandlung von Kontrollpflanzen mit ALS-inhibierenden Herbiziden (Höfgen et al., Plant Physiology 107(1995), 469-477).

Aufgabe der vorliegenden Erfindung war es zu belegen, daß Aspartat-Carbamyltransferase in Pflanzen ein geeignetes herbizides Target ist, sowie die Herstellung eines effizienten und einfachen Aspartat-Carbamyltransferase Testsystems für die Durchführung von Inhibitor-Enzym-Bindungsstudien.

Die Aufgabe wurde gelöst durch Isolierung von DNA-Sequenzen, die für das pflanzliche Enzym Aspartat-Carbamyltransferase kodieren, der Herstellung von Antisensekonstrukten der pflanzlichen Aspartat-Carbamyltransferase und deren Expression in Pflanzen, sowie der funktionellen Expression der pflanzlichen Aspartat-Carbamyltransferase in bakteriellen oder eukaryontischen Zellen.

Zur Lösung der Aufgabe wurde eine cDNA codierend für pflanzliche Aspartat-Carbamyltransferase aus Kartoffel sowie zwei partielle cDNAs aus Physcomitrella patens isoliert und sequenziert, siehe Beispiel 1, 2.1 und 2.2 bzw. Sequenzprotokoll SEQ-ID No. 1, SEQ-ID No. 3 und SEQ-ID No. 5.

Kartoffelpflanzen, die ein Antisensekonstrukt der Aspartat-Carbamyltransferase tragen, wurden näher charakterisiert. Die Pflanzen zeigen in unterschiedlichem Maße eine Wachstumsretardierung. Die transgenen Linien, sowie die Nachkommen der 1. und 2. Generation, wiesen ein verringertes Wachstum in Erde auf. In Pflanzen mit verringertem Wachstum konnte eine im Vergleich zum Wildtyp reduzierte Aspartat-Carbamyltransferase RNA-Menge in der Northern-Hybridisierung detektiert werden, siehe Beispiel 5. Ferner konnte durch Messung der Enzymaktivität eine im Vergleich mit Wildtyppflanzen verringerte Menge der Aspartat-Carbamyltransferase Aktivität in den transgenen Linien detektiert werden. Das Expressionsniveau sowie die Reduktion der Aspartat-Carbamyltransferase Aktivität korrelieren mit der Wachstumsretardierung. Obwohl in Kartoffelpflanzen mit hoher Wahrscheinlichkeit mehrere Isoformen der Aspartat-Carbamyltransferase vorkommen, wurde überraschenderweise gefunden, daß durch Einbringen eines Aspartat-Carbamyltransferase-Antisensekonstruktes eine Verringerung des Wachstums der Pflanze zu beobachten ist. Dieser klare Zusammenhang weist Aspartat-Carbamyltransferase erstmals eindeutig als geeignetes Zielprotein (Target) für herbizide Wirkstoffe aus.

Ein weiterer Gegenstand der Erfindung betrifft Verfahren zur Identifizierung von Inhibitoren der pflanzlichen Aspartat-Carbamyltransferase durch Hochdurchsatzmethoden. Die Erfindung betrifft daher die funktionale Expression pflanzlicher Aspartat-Carbamyltransferase insbesondere von Aspartat-Carbamyltransferase aus Kartoffel und Physcomitrella patens in geeigneten Expressionssystemen sowie die Verwendung der so hergestellten Enzyme in einem *in vitro* Testsystem zur Messung der Aspartat-Carbamyltransferase-Aktivität.

Um effiziente Hemmstoffe der pflanzlichen Aspartat-Carbamyl-transferase finden zu können, ist es notwendig, geeignete Testsysteme, mit denen Inhibitor-Enzym-Bindungsstudien durchgeführt werden können, zur Verfügung zu stellen. Hierzu wird beispielsweise die cDNA-Sequenz der Aspartat-Carbamyltransferase der cDNA-Sequenz der Aspartat-Carbamyltransferase aus Kartoffel oder Physcomitrella patens in einen Expressionsvektor (pQE, Qiagen) kloniert und in E. coli überexprimiert.

Alternativ kann jedoch die Expressionskassette enthaltend eine DNA-Sequenz der SEQ-ID No. 1, SEQ-ID No. 3 oder SEQ-ID No. 5 beispielsweise in anderen Bakterien, in Hefen, Pilzen, Algen, Pflanzenzellen, Insektenzellen oder Säugetierzellen exprimiert werden.

Weiterer Gegenstand der Erfindung ist die Verwendung von DNA-Sequenzen, die eine Sequenz von 80 bis 100 % zu von SEQ-ID No. 1, SEQ-ID No. 3 oder SEQ-ID No. 5 besitzen und die für ein Protein kodieren, das die biologische Aktivität einer Aspartat-Carbamyltransferase besitzt.

Das mit Hilfe einer Expressionskassette exprimierte pflanzliche Aspartat-Carbamyltransferase Protein eignet sich besonders zur Auffindung von für die Aspartat-Carbamyltransferase spezifischen Hemmstoffen.

Dazu kann die pflanzliche Aspartat-Carbamyltransferase beispielsweise in einem Enzymtest eingesetzt werden, bei dem die Aktivität der Aspartat-Carbamyltransferase in An- und Abwesenheit des zu testenden Wirkstoffs ermittelt wird. Aus dem Vergleich der beiden Aktivitätsbestimmungen läßt sich eine qualitative und quantitative Aussage über das Hemmverhalten des zu testenden Wirkstoffes machen, siehe Beispiel 9.

Mit Hilfe des erfindungsgemäßen Testsystems kann eine Vielzahl von chemischen Verbindungen schnell und einfach auf herbizide Eigenschaften überprüft werden. Das Verfahren gestattet es, reproduzierbar aus einer großen Anzahl von Substanzen gezielt solche mit großer Wirkstärke auszuwählen, um mit diesen Substanzen anschließend weitere, dem Fachmann geläufige vertiefte Prüfungen durchzuführen.

Weiterer Gegenstand der Erfindung ist ein Verfahren zur Identifizierung von Inhibitoren pflanzlicher Aspartat-Carbamyltransferase mit potentiell herbizider Wirkung, indem man das Gen einer pflanzlichen Aspartat-Carbamyltransferase kloniert, in einer geeigneten Expressionskassette - beispielsweise in Insektenzellen - zur Überexpression bringt, die Zellen öffnet und den Zellextrakt direkt bzw. nach Anreicherung oder Isolierung des Enzyms Aspartat-Carbamyltransferase in einem Testsystem zur Messung der Enzymaktivität in Gegenwart von niedermolekularen chemischen Verbindungen einsetzt.

Weiterer Gegenstand der Erfindung ist ein Verfahren zur Identifizierung von Substanzen mit herbizider Wirkung, die die Aspartat-Carbamyltransferase Aktivität in Pflanzen hemmen, bestehend aus
a) der Herstellung von transgenen Pflanzen, Pflanzengeweben, oder Pflanzenzellen, die eine zusätzliche DNA-Sequenz codierend für ein Enzym mit Aspartat-Carbamyltransferase Aktivität enthalten und in der Lage sind eine enzymatisch aktive Aspartat-Carbamyltransferase überzuexprimieren;
b) das Aufbringen einer Substanz auf transgene Pflanzen, Pflanzenzellen, Pflanzengewebe oder Pflanzenteile sowie auf nicht-transformierten Pflanzen, Pflanzenzellen, Pflanzengewebe oder Pflanzenteile;
c) das Bestimmen des Wachstums oder der Überlebensfähigkeit der transgenen und der nicht-transformierten Pflanzen, Pflanzenzellen, Pflanzengewebe oder Pflanzenteile nach der Aufbringung der chemischen Substanz; und
d) dem Vergleich des Wachstums oder der Überlebensfähigkeit der transgenen und der nicht-transformierten Pflanzen, Pflanzenzellen, Pflanzengewebe oder Pflanzenteile nach der Aufbringung der chemischen Substanz;
wobei die Unterdrückung des Wachstums oder der Überlebensfähigkeit der nicht-transformierten Pflanzen, Pflanzenzellen, Pflanzengewebe oder Pflanzenteile ohne jedoch das Wachstum oder die Überlebensfähigkeit der transgenen Pflanzen, Pflanzenzellen, Pflanzengewebe oder Pflanzenteile stark zu unterdrücken, belegt, daß die Substanz aus b) herbizide Aktivität zeigt und die Aspartat-Carbamyltransferase Enzymaktivität in Pflanzen inhibiert.

Inhibitoren der Aspartat-Carbamyltransferase mit herbizider Wirkung können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, an denen sie unerwünscht sind. Ob die mit Hilfe des erfindungsgemäßen Testsystems gefundenen Wirkstoffe als totale oder selektive Herbizide wirken, hängt unter anderem von der angewandten Menge ab.

Inhibitoren der Aspartat-Carbamyltransferase mit herbizider Wirkung können beispielsweise gegen folgende Unkräuter verwendet werden:
Dikotyle Unkräuter der Gattungen:
   Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Monokotyle Unkräuter der Gattungen:
   Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristyslis, Sagittaria, Eleocharis, Scirpus, Paspalum, Ischaemum, Sphenoclea, Dactyloctenium, Agrostis, Alopecurus, Apera.

Gegenstand der Erfindung ist auch die Verwendung von Expressionskassetten, deren Sequenz für eine Aspartat-Carbamyltransferase aus Kartoffel oder Physcomitrella patens kodieren zur Herstellung eines Testsystems zum Auffinden von Verbindungen mit herbizider Wirkung. Die Nukleinsäuresequenz kann dabei z.B. eine DNA- oder eine cDNA-Sequenz sein.

Derartige Expressionskassetten beinhalten außerdem regulative Nukleinsäuresequenzen, welche die Expression der kodierenden Sequenz in der Wirtszelle steuern. Gemäß einer bevorzugten Ausführungsform umfaßt eine erfindungsgemäße Expressionskassette stromaufwärts, d.h. am 5'-Ende der kodierenden Sequenz, einen Promotor und stromabwärts, d.h. am 3'-Ende, ein Polyadenylierungssignal und gegebenenfalls weitere regulatorische Elemente, welche mit der dazwischenliegenden kodierenden Sequenz für das Aspartat-Carbamyltransferase Gen operativ verknüpft sind. Unter einer operativen Verknüpfung versteht man die sequenzielle Anordnung von Promotor, kodierender Sequenz, Terminator und ggf. weiterer regulativer Elemente derart, daß jedes der regulativen Elemente seine Funktion bei der Expression der kodierenden Sequenz bestimmungsgemäß erfüllen kann.

Die Herstellung einer derartigen Expressionskassette erfolgt durch Fusion eines geeigneten Promotors mit einer geeigneten Aspartat-Carbamyltransferase DNA Sequenz und einem Polyadenylierungssignal nach gängigen Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley-Interscience (1987) beschrieben sind.

Gegenstand der Erfindung ist die Verwendung von DNA-Sequenzen, die für ein Aspartat-Carbamyltransferase Gen kodieren und die bezogen auf die Gesamtlänge der DNA-Sequenz eine Sequenzidentität mit der DNA-Sequenz SEQ-ID No. 1, SEQ-ID No. 3 oder SEQ-ID No. 5 von 80 bis 100 % aufweisen.

Funktionell äquivalente Sequenzen, die für ein Aspartat-Carbamyltransferase Gen kodieren, sind solche Sequenzen, welche trotz abweichender Nukleotidsequenz noch die gewünschten Funktionen besitzen. Funktionelle Äquivalente umfassen somit natürlich vorkommende Varianten der hierin beschriebenen Sequenzen sowie künstliche, z.B. durch chemische Synthese erhaltene, an den Kodon-Gebrauch einer Pflanze angepaßte, künstliche Nukleotid-Sequenzen.

Unter einem funktionellen Äquivalent versteht man insbesondere auch natürliche oder künstliche Mutationen einer ursprünglich isolierten für eine Aspartat-Carbamyltransferase kodierende Sequenz, welche weiterhin die gewünschte Funktion zeigt. Mutationen umfassen Substitutionen, Additionen, Deletionen, Vertauschungen oder Insertionen eines oder mehrerer Nukleotidreste. Somit werden beispielsweise auch solche Nukleotidsequenzen durch die vorliegende Erfindung mit umfaßt, welche man durch Modifikation dieser Nukleotidsequenz erhält. Ziel einer solchen Modifikation kann z.B. die weitere Eingrenzung der darin enthaltenen kodierenden Sequenz oder z.B. auch die Einfügung weiterer Restriktionsenzym-Schnittstellen sein.

Funktionelle Äquivalente sind auch solche Varianten, deren Funktion, verglichen mit dem Ausgangsgen bzw. Genfragment, abgeschwächt oder verstärkt ist.

Die Expressionskassette kann darüberhinaus auch zur Transformation von Bakterien, Cyanobakterien, Hefen, filamentösen Pilzen und Algen und eukaryontischen Zellen (z.B. Insektenzellen) mit dem Ziel der Herstellung von ausreichenden Mengen des Enzyms Aspartat-Carbamyltransferase eingesetzt werden.

Weiterer Gegenstand der Erfindung ist die Verwendung eines Proteins aus Kartoffel oder Physcomitrella patens gekennzeichnet durch die Aminosäuresequenz SEQ-ID No. 2 bzw. SEQ-ID No. 4 bzw. SEQ-ID No. 6 mit Aspartat-Carbamyltransferase Aktivität zur Herstellung eines Testsystems zum Auffinden von Verbindungen mit herbizider Wirkung.

Besonders bevorzugt ist die Verwendung pflanzlicher Proteine mit Aspartat-Carbamyltransferase Aktivität mit einer Aminosäuresequenzidentität zu der Aspartat-Carbamyltransferase aus Kartoffel oder Physcomitrella patens mit den Sequenzen SEQ-ID No. 2 bzw. SEQ-ID No. 4 bzw. SEQ-ID No. 6 von 80 - 100 % zur Herstellung eines Testsystems zum Auffinden von Verbindungen mit herbizider Wirkung.

Erfindungsgemäß umfaßt sind auch Isoenzyme der Aspartat-Carbamyltransferase. So codieren die Gene aus *Physcomitrella patens* mit SEQ-ID No. 3 und SEQ-ID No. 5 für zwei ATCase Isoenzyme (SEQ-ID No. 4 und 6), die wahrscheinlich in unterschiedlichen Entwicklungsstadien in Moos exprimiert werden.

Unter Isoenzyme sind Isoformen von Enzymen mit gleicher oder vergleichbarer Substrat- und Wirkungsspezifität zu verstehen, die jedoch eine unterschiedliche Primärstruktur aufweisen.

Die Wirksamkeit der Expression des transgen exprimierten Aspartat-Carbamyltransferase Gens kann beispielsweise in vitro durch Sproßmeristemvermehrung oder durch einen Keimungstest ermittelt werden. Zudem kann eine in Art und Höhe veränderte Expression des Aspartat-Carbamyltransferase Gens und deren Auswirkung auf die Resistenz gegenüber Hemmstoffen der Aspartat-Carbamyltransferase an Testpflanzen in Gewächshausversuchen getestet werden.

Beschrieben sind außerdem transgene Pflanzen, transformiert mit einer Expressionskassette, enthaltend die DNA-Sequenz SEQ-ID No. 1, SEQ-ID No. 3 oder SEQ-ID No 5 die durch zusätzliche Expression der DNA-Sequenz SEQ-ID No. 1, SEQ-ID No. 3 oder SEQ-ID No. 5 tolerant gegenüber Inhibitoren der Aspartat-Carbamyltransferase geworden sind, sowie transgene Zellen, Gewebe, Teile und Vermehrungsgut solcher Pflanzen. Besonders bevorzugt sind dabei transgene Kulturpflanzen, wie z.B. Gerste, Weizen, Roggen, Mais, Soja, Reis, Baumwolle, Zuckerrübe, Canola, Sonnenblume, Flachs, Hanf, Kartoffel, Tabak, Tomate, Raps, Alfalfa, Salat und die verschiedenen Baum-, Nuß- und Weinspezies, sowie Leguminosen.

Insbesondere bevorzugt sind Sequenzen, die ein Targeting in den Apoplasten, in Plastiden, die Vakuole, das Mitochondrium, das Endoplasmatische Retikulum (ER) oder durch ein Fehlen entsprechender operativer Sequenzen einen Verbleib im Kompartiment des Entstehens, dem Zytosol, gewährleisten (Kermode, Crit. Rev. Plant Sci. 15, 4 (1996), 285-423).

Beispielhaft kann die pflanzliche Expressionskassette in den PflanzenTransformationsvektor pBinAR eingebaut werden.

Als Promotoren der Expressionskassette ist grundsätzlich jeder Promotor geeignet, der die Expression von Fremdgenen in Pflanzen steuern kann. Vorzugsweise verwendet man insbesondere einen pflanzlichen Promotor oder einen Promotor, der einem Pflanzenvirus entstammt. Insbesondere bevorzugt ist der CaMV 35S-Promotor aus dem Blumenkohl-Mosaik-Virus (Franck et al., Cell 21 (1980), 285-294). Dieser Promotor enthält unterschiedliche Erkennungssequenzen für transkriptionale Effektoren, die in ihrer Gesamtheit zu einer permanenten und konstitutiven Expression des eingeführten Gens führen (Benfey et al., EMBO J., 8 (1989), 2195-2202).

Die Expressionskassette kann auch einen chemisch induzierbaren Promotor enthalten, durch den die Expression des exogenen Aspartat-Carbamyltransferase Gens in der Pflanze zu einem bestimmten Zeitpunkt gesteuert werden kann. Derartige Promotoren wie z.B. der PRP1-Promotor (Ward et al., Plant.Mol. Biol. 22(1993), 361-366), ein durch Salizylsäure induzierbarer Promotor (WO 95/19443), ein durch Benzenesufonamid-induzierbarer (EP 388186), ein durch Tetrazyklininduzierbarer (Gatz et al., Plant J. 2(1992), 397-404), ein durch Abscisinsäure-induzierbarer (EP0335528) bzw. ein durch Ethanol- oder Cyclohexanon-induzierbarer (WO 93/21334) Promotor sind in der Literatur beschrieben und können u.a. verwendet werden.

Weiterhin sind insbesonders solche Promotoren bevorzugt, die die Expression in Geweben oder Pflanzenteilen sicherstellen, in denen die Biosynthese von Purinen bzw. deren Vorstufen stattfindet. Insbesondere zu nennen sind Promotoren, die eine blattspezifische Expression gewährleisten. Zu nennen sind der Promotor der cytosolischen FBPase aus Kartoffel oder der ST-LSI Promotor aus Kartoffel (Stockhaus et al., EMBO J., 8 (1989) 2445-245).

Mit Hilfe eines samenspezifischen Promotors kann ein Fremdprotein stabil bis zu einem Anteil von 0,67% des gesamten löslichen Samenproteins in den Samen transgener Tabakpflanzen exprimiert werden (Fiedler und Conrad, Bio/Technology 10 (1995), 1090-1094). Die erfindungsgemäße Expressionskassette kann daher beispielsweise einen samenspezifischen Promotor (bevorzugt den Phaseolin-Promotor, den USP- oder LEB4-Promotor), das LEB4-Signalpeptid, das zu exprimierende Gen und ein ER-Retentionssignal enthalten.

Die inserierte Nukleotid-Sequenz kodierend für eine Aspartat-Carbamyltransferase kann synthetisch hergestellt oder natürlich gewonnen sein oder eine Mischung aus synthetischen und natürlichen DNA-Bestandteilen enthalten. Im allgemeinen werden synthetische Nukleotid-Sequenzen mit Kodons erzeugt, die von Pflanzen bevorzugt werden. Diese von Pflanzen bevorzugten Kodons können aus Kodons mit der höchsten Proteinhäufigkeit bestimmt werden, die in den meisten interessanten Pflanzenspezies exprimiert werden. Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente miteinander können an die Fragmente Adaptoren oder Linker angesetzt werden.

Außerdem sind artifizielle DNA-Sequenzen geeignet, solange sie, wie oben beispielsweise beschrieben, die gewünschte Eigenschaft der Expression des Aspartat-Carbamyltransferase Gens vermitteln. Solche artifiziellen DNA-Sequenzen können beispielsweise durch Rückübersetzung mittels Molecular Modelling konstruierter Proteine, die Aspartat-Carbamyltransferase Aktivität aufweisen, oder durch in vitro-Selektion ermittelt werden. Besonders geeignet sind kodierende DNA-Sequenzen, die durch Rückübersetzung einer Polypeptidsequenz gemäß der für die Wirtspflanze spezifischen Kodon-Nutzung erhalten wurden. Die spezifische Kodon-Nutzung kann ein mit pflanzengenetischen Methoden vertrauter Fachmann durch Computerauswertungen anderer, bekannter Gene der zu transformierenden Pflanze leicht ermitteln.

Als weitere geeignete äquivalente Nukleinsäure-Sequenzen sind zu nennen Sequenzen, welche für Fusionsproteine kodieren, wobei Bestandteil des Fusionsproteins ein pflanzliches Aspartat-Carbamyltransferase Polypeptid ist. Der zweite Teil des Fusionsproteins kann z.B. ein weiteres Polypeptid mit enzymatischer Aktivität sein oder eine antigene Polypeptidsequenz mit deren Hilfe ein Nachweis auf Aspartat-Carbamyltransferase Expression möglich ist (z.B. myc-tag oder his-tag). Bevorzugt handelt es sich dabei jedoch um eine regulative Proteinsequenz, wie z.B. ein Signal- oder Transitpeptid, das das Aspartat-Carbamyltransferase Protein an den gewünschten Wirkort leitet.

Zweckmäßigerweise sollten die Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der erfindungsgemäße Promotor kann sowohl nativ bzw. homolog als auch fremdartig bzw. heterolog zur Wirtspflanze sein. Die erfindungsgemäße Expressionskassette beinhaltet in der 5'-3'-Transkriptionsrichtung den erfindungsgemäßen Promotor, eine beliebige Sequenz und eine Region für die transkriptionale Termination. Verschiedene Terminationsbereiche sind gegeneinander beliebig austauschbar.

Ferner können Manipulationen, die passende Restriktionsschnittstellen bereitstellen oder die überflüssige DNA oder Restriktionsschnittstellen entfernen, eingesetzt werden. Wo Insertionen, Deletionen oder Substitutionen wie z.B. Transitionen und Transversionen in Frage kommen, können in *vitro*-Mutagenese, "primerrepair", Restriktion oder Ligation verwendet werden. Bei geeigneten Manipulationen, wie z.B. Restriktion, "chewing-back" oder Auffüllen von Überhängen für "bluntends", können komplementäre Enden der Fragmente für die Ligation zur Verfügung gestellt werden.

Bevorzugte Polyadenylierungssignale sind pflanzliche Polyadenylierungssignale, vorzugsweise solche, die im wesentlichen T-DNA-Polyadenylierungssignale aus Agrobacterium tumefaciens, insbesondere des Gens 3 der T-DNA (Octopin Synthase) des Ti-Plasmids pTiACH5 entsprechen (Gielen et al., EMBO J., 3 (1984), 835) oder funktionelle Äquivalente.

Zur Transformation einer Wirtspflanze mit einer für eine Aspartat-Carbamyltransferase kodierenden DNA wird eine Expressionskassette als Insertion in einen rekombinanten Vektor eingebaut, dessen Vektor-DNA zusätzliche funktionelle Regulationssignale, beispielsweise Sequenzen für Replikation oder Integration enthält. Geeignete Vektoren sind unter anderem in "Methods in Plant Molecular Biology and Biotechnology" (CRC Press, Kapitel 6/7, 71-119) beschrieben.

Die Übertragung von Fremdgenen in das Genom einer Pflanze wird als Transformation bezeichnet. Es werden dabei die beschriebenen Methoden zur Transformation und Regeneration von Pflanzen aus Pflanzengeweben oder Pflanzenzellen zur transienten oder stabilen Transformation genutzt. Geeignete Methoden sind die Protoplastentransformation durch Polyethylenglykol-induzierte DNA-Aufnahme, der biolistische Ansatz mit der Genkanone, die Elektroporation, die Inkubation trockener Embryonen in DNA-haltiger Lösung, die Mikroinjektion und der durch Agrobacterium vermittelte Gentransfer. Die genannten Verfahren sind beispielsweise in B. Jenes et al., Techniques for Gene Transfer, in: Transgenic Plants, Vol. 1, Engineering and Utilization, herausgegeben von S.D. Kung und R. Wu, Academic Press (1993), 128-143 sowie in Potrykus Annu. Rev. Plant Physiol.Plant Molec.Biol. 42 (1991), 205-225 beschrieben. Vorzugsweise wird das zu exprimierende Konstrukt in einen Vektor kloniert, der geeignet ist, Agrobacterium tumefaciens zu transformieren, beispielsweise pBin19 (Bevan et al., Nucl. Acids Res. 12 (1984), 8711).

Mit einer Expressionskassette transformierte Agrobakterien können ebenfalls in bekannter Weise zur Transformation von Pflanzen, insbesondere von Kulturpflanzen, wie Getreide, Mais, Soja, Reis, Baumwolle, Zuckerrübe, Canola, Sonnenblume, Flachs, Hanf, Kartoffel, Tabak, Tomate, Raps, Alfalfa, Salat und den verschiedenen Baum-, Nuß- und Weinspezies sowie Leguminosen verwendet werden, z.B. indem verwundete Blätter oder Blattstücke in einer Agrobakterienlösung gebadet und anschließend in geeigneten Medien kultiviert werden.

Der Biosytheseort von Pyrimidinen ist im allgemeinen das Blattgewebe, so daß eine blattspezifische Expression des Aspartat-Carbamyltransferase Gens sinnvoll ist. Es ist jedoch naheliegend, daß die PyrimidinBiosynthese nicht auf das Blattgewebe beschränkt sein muß, sondern auch in allen übrigen Teilen der Pflanze - beispielsweise in fetthaltigen Samen - gewebespezifisch erfolgen kann.

Darüberhinaus ist eine konstitutive Expression des exogenen Aspartat-Carbamyltransferase Gens von Vorteil. Andererseits kann aber auch eine induzierbare Expression wünschenswert erscheinen.

Unter Verwendung der oben zitierten Rekombinations- und Klonierungstechniken können die Expressionskassetten in geeignete Vektoren kloniert werden, die ihre Vermehrung, beispielsweise in E. coli, ermöglichen. Geeignete Klonierungsvektoren sind u.a. pBR332, pUC-Serien, M13mp-Serien und pACYC184. Besonders geeignet sind binäre Vektoren, die sowohl in E. coli als auch in Agrobakterien replizieren können.

Weiterhin wird die Verwendung einer Expressionskassette zur Transformation von Pflanzen, Pflanzenzellen, -geweben oder Pflanzenteilen beschrieben. Vorzugsweise ist Ziel der Verwendung die Erhöhung des Aspartat-Carbamyltransferase Gehaltes in der Pflanze.

Dabei kann je nach Wahl des Promotors die Expression spezifisch in den Blättern, in den Samen oder anderen Teilen der Pflanze erfolgen. Solche transgenen Pflanzen, deren Vermehrungsgut sowie deren Pflanzenzellen, -gewebe oder -teile sind ein weiterer Gegenstand der vorliegenden Erfindung.

Die Erfindung wird durch die nun folgenden Beispiele erläutert, ist aber nicht auf diese beschränkt.

Gentechnische Verfahren, die den Ausführungsbeispielen zugrunde liegen:

### Allgemeine Klonierungsverfahren

Klonierungsverfahren wie z.B.: Restriktionsspaltungen, DNA-Isolierung, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von E. coli Zellen, Anzucht von Bakterien, Sequenzanalyse rekombinanter DNA wurden nach Sambrook et al.,Cold Spring Harbor Laboratory Press (1989); ISBN 0-87969-309-6 beschrieben durchgeführt. Die Transformation von Agrobacterium tumefaciens wurde entsprechend der Methode von Höfgen und Willmitzer (Nucl. Acid Res. 16(1988), 9877) ausgeführt. Die Anzucht der Agrobacterien erfolgte in YEB Medium (Vervliet et al., Gen. Virol. 26(1975), 33).

### Sequenzanalyse rekombinanter DNA

Die Sequenzierung rekombinanter DNA-Moleküle erfolgte mit einem Laserfluoreszenz-DNA-Sequenzierer der Firma ABI nach der Methode von Sanger (Sanger et al., Proc. Natl. Acad. Sci. USA, 74(1977), 5463-5467). Fragmente resultierend aus einer Polymerase Kettenreaktion wurden zur Vermeidung von Polymerasefehlern in zu exprimierenden Konstrukten sequenziert und überprüft.

### Analyse von Gesamt-RNA aus pflanzlichen Geweben

Gesamt-RNA aus pflanzlichen Geweben wurde wie bei Logemann et al., Anal. Biochem. 163(1987), 21 aus Blättern isoliert. Für die Analyse wurden jeweils 20 Microgramm RNA in einem Formaldehyd-haltigen 1,5%igen Agarosegel aufgetrennt und auf Duralon UV Membranen (Stratagene) überführt. Zum Nachweis spezifischer Transkripte wurden nach Herstellerangaben Digoxygenin-markierte, strangspezifische Sonden hergestellt (DIG RNA Labeling Kit SP6/T7, Roche Diagnostics) und zur Hybridisierung verwendet (DIG EasyHyb, Roche Diagnostics). Anschließend wurden die Membranen 3 x 20 Min in Waschpuffer (2x SSC, 0,1% SDS) bei 60°C gewaschen. Die Detektion erfolgte mittels des DIG-Detektionssystems von Roche Diagnostics mit CDP-Star als Substrat durch Lumineszenz und Exposition auf Hyperfilm ECL (Amersham).

### Western-Blot Analyse

Blattproben wurden in Probenpuffer für die SDS-Polyacrylamidgelelektrophorese (Sambrook et al. 1989, Cold Spring Harbor Laboratory Press: ISBN 0-87969-309-6) im Verhältnis 1:10 gemörsert und 10 Minuten bei 95 °C denaturiert. Gleiche Mengen der Extrakte wurden durch SDS-Polyacrylamid-Gelelektrophorese (PAGE) aufgetrennt und auf PVDF-Membranen transferiert. Die Membranen wurden 1 Stunde in 5% Blocking-Lsg. (Blotto, BioRad) bei Raumtemperatur inkubiert. Es schlossen sich folgende Inkubationen an:
Antikörperinkubation: Verdünnung 1:2000 des Antiserums für 1 Stunde bei RT in 2,5% Blocking-Lsg.
Waschen: 1 x kurz mit TBS (20 mM Tris HCl und 500 mM NaCl pH 7,5), 1 x 15 Minuten TBST (TBS mit 0,1 % (v/v) Tween 20), 1 x 15 Minuten TBS.
Antikörperinkubation: Anti-rabbit IgG mit alkalischer Phosphatase gekoppelt (BioRad) in einer Verdünnung von 1:2000 in 2,5% Blocking-Lsg. für 1 Stunde bei RT.
Waschen: 1 x kurz mit TBS (20 mM Tris HCI und 500 mM NaCl pH 7,5), 1 x 15 Minuten
Detektion: Inkubation in 100 mM Tris-HCI, pH 9,5; 100 mM NaCl; 5 mM MgCl₂ mit BCIP (0,167 mg / ml) und NBT (0,333 mg / ml).

Die verwendeten Chemikalien wurden, sofern nicht anders erwähnt, in p.a. Qualität von den Firmen Fluka (Neu-Ulm), Merck (Darmstadt), Roth (Karlsruhe), Serva (Heidelberg) sowie Sigma (Deisenhofen) bezogen. Lösungen wurden mit aufbereitetem, pyrogenfreiem Wasser, im weiteren Text als H₂O bezeichnet, aus einer Milli-Q Water System Wasseraufbereitungsanlage (Millipore, Eschborn) angesetzt. Restriktionsendonukleasen, DNA-modifizierende Enzyme und molekularbiologische Kits wurden von den Firmen AGS (Heidelberg), Amersham (Braunschweig), Biometra (Göttingen), Roche (Mannheim), Genomed (Bad Oeynnhausen), New England Biolabs (Schwalbach/Taunus), Novagen (Madison, Wisconsin, USA), Perkin-Elmer (Weiterstadt), Pharmacia (Freiburg) Qiagen (Hilden) und Stratagene (Heidelberg) bezogen. Sie wurden, soweit nicht anders erwähnt, nach Herstellerangaben verwendet.

### Beispiel 1

### Isolierung einer cDNA codierend für eine funktionelle ATCase aus Kartoffel

Ein Klon codierend für eine ATCase aus Kartoffel wurde über funktionelle Komplementation einer *E.coli* pyrB Mutante CGSC#6852 des *E*. *coli* Genetic Stock Centers isoliert (RBP3b). Die Komplementation erfolgte durch Elektrotransformation mit 5*10⁵ cfu einer cDNA Bank in dem Vektorplasmid pBS SK-. Die zugrunde liegende Lambda ZAPII Bank (Stratagene) wurde nach Standardvorschriften mit EcoRI/Notl Linkern hergestellt. Die RNA-Matrize zur Herstellung der cDNA-Bank wurde aus sink-Blättern von Kartoffel (kleiner 1 cm, 10 Wochen alte Kartoffelpflanzen aus dem Gewächshaus) isoliert.

Die transformierten Bakterien wurden auf M9 Minimalmedium plattiert (Sambrook et al., 1989), das zusätzlich Methionin (20 mg/l), Thiamin (100 mg/l), Ampicillin (100 mg/l) und IPTG (2.5 mM) enthielt. Das Plasmid eines komplementierenden Klons (benannt pRBP3b) wurde isoliert und sequenziert. Die cDNA codiert auf 1344 Nukleotiden ein Protein von 412 Aminosäuren (SEQ-ID No. 1 und 2). Ein Sequenzvergleich der Amninosäuresequenz mit der ATCase aus Erbse (Genbank Accession No. M96981) läßt vermuten, daß RBP3b eine cDNA voller Länge darstellt. Aminosäure 36 stellt wahrscheinlich das Startmethionin dar. Das abgeleitete Protein ist zu 83,9% ähnlich zur ATCase aus *Arabidopsis thaliana.* Die Ähnlichkeit zu ATCasen aus *E.coli* und Mensch beträgt 63,0% bzw. 65,6%. Die Nukleotidsequenz ist zum Gen aus *Arabidopsis thaliana* zu 75,4%, zu dem Gen aus *E.coli* und zur cDNA aus Mensch zu 54,4% bzw. 66,4% identisch.

### Beispiel 2.1.

### Isolierung einer cDNA codierend für eine ATCase aus Moos

Aus mRNA von *Physcomitrella patens* Protonemata wurde doppelsträngige cDNA erzeugt und zur Herstellung einer cDNA-Bank im Vektor pBluescript SKII verwendet (lambda ZAP II RI Library construction Kit, Stratagene). Einzelne Klone dieser Bank wurden ansequenziert. Die 3'-seitige Sequenz des Klons pp001057045r wies deutliche Homologie zu ATCase aus Pflanzen auf. Der Klon pp001 057045r wurde vollständig sequenziert. Er codiert auf einer partiellen cDNA von 617 Basenpaaren für ein Polypeptid von 118 Aminosäuren (SEQ-ID No. 3 und 4). Auf Aminosäureebene ist pp001057045r zu 72,9 % identisch zur ATCase aus *Arabidopsis thaliana* und zu 49,1 % identisch zur ATCase aus *E.coli.* Die Nukleotidsequenz ist zum Gen aus *Arabidopsis thaliana* zu 75,4 % und zum Gen aus E.coli und Mensch zu 54,8 % identisch.

### Beispiel 2.2

### Isolierung einer cDNA codierend für eine weitere ATCase aus Moos (Isoenzym)

Aus mRNA von *Physcomitrella patens* Gametophoren wurde doppelsträngige cDNA erzeugt und von *Physcomitrella patens* cDNA aus Protonema subtahiert. Die erhaltene cDNA wurde zur Herstellung einer Plasmid cDNA-Bank im Vektor pUC19(Roche, Mannheim) verwendet.

Einzelne Klone dieser Bank wurden ansequenziert. Die 3'-seitige Sequenz des Klons c_pp015013185r wies deutliche Homologie zu ATCase aus Pflanzen auf. Der Klon c_pp015013185r wurde vollständig sequenziert. Die partielle cDNA von 1251 Basenpaaren codiert für ein Polypeptid von 310 Aminosäuren (SEQ-ID No. 5 und 6). Die Nukleotidsequenz von c_pp015013185r ist zu 69% identisch zu dem Gen aus Arabidopsis thaliana (Genbank Accession No. X71843). Das von c_pp015013185r codierte Protein ist zu 79,7% identisch mit ATCase aus Arabidopsis. Die Identität zu dem Klon pp001057045r aus Moos beträgt 74,5% auf Nukleotidsequenzebene und 81,9% auf Aminosäuresequenzebene. Diese beiden Klone aus Moos codieren für zwei ATCase Isoenzyme, die wahrscheinlich in unterschiedlichen Entwicklungsstadien in Moos exprimiert werden.

### Beispiel 3

### Herstellung eines Pflanzentransformationsvektors

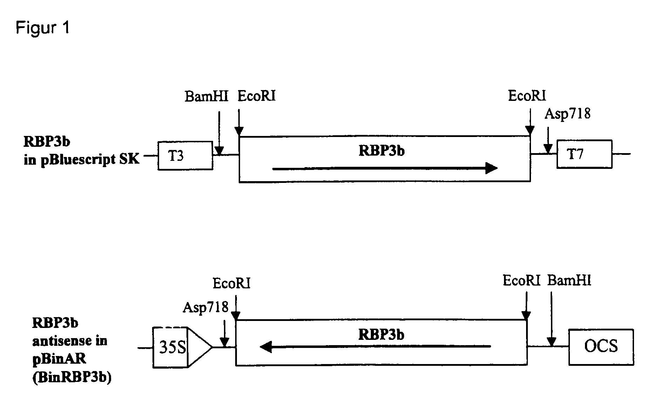

Ein RBP3b cDNA-Fragment von 1494bp, enthaltend die RBP3b cDNA wurde durch Spaltung mit Asp718 und BamHI erhalten und in den ebenso gespaltenen Vektor BinAR (Höfgen und Willmitzer, Plant Science 66 (1990), 221-230) ligiert. Das resutierende Konstrukt BinRBP3b enthält die RBP3b cDNA in Antisenseorientierung zum 35S-Promotor des Blumenkohl-Mosaikvirus (Abbildung 1).

### Beispiel 4

### Herstellung transgener Kartoffelpflanzen

Zur Transformation von Kartoffelpflanzen (Cv. Desirée) wurde das Konstrukt BinRBP3b zunächst in *Agrobacterium tumefaciens* C58C1:pGV2260 transformiert (Deblaere et al., Nucl. Acids. Res. 13(1984), 4777-4788). Zur Transformation von Kartoffel nach Rocha-Sosa et al., EMBO J., 8(1988), 23-29, wurde eine 1:50 Verdünnung einer Übernachtkultur einer positiv transformierten Agrobakterienkolonie in Murashige-Skoog Medium (Physiol. Plant. 15(1962), 473) benutzt. Blattscheiben steriler Pflanzen (zu je ca. 1 cm²) wurden in einer Petrischale mit einer 1:50 Agrobakterienverdünnung für 5-10 Minuten inkubiert. Es folgte eine 2-tägige Inkubation in Dunkelheit bei 20□C auf MS-Medium. Die Kultivierung wurde anschließend mit 16 Stunden Licht/8 Stunden Dunkelheit weitergeführt. Im wöchentlichem Rhythmus wurde auf MS-Medium mit 500 mg/l Claforan (Cefotaxime-Natrium), 50 mg/l Kanamycin und Pflanzenhormonen (Rocha-Sosa et al., EMBO J., 8(1989); 23-29) und 16 g/l Glukose zur Sprossinduktion umgesetzt. Wachsende Sprosse wurden auf MS-Medium mit 2% Saccharose, 250 mg/l Claforan und 0,8% Bacto-Agar überführt.

Regenerierte Sprosse werden auf MS-Medium mit 20 g/l Saccharose, mit Kanamycin und Claforan erhalten, nach Bewurzelung in Erde überführt und nach Kultivierung für zwei Wochen in einer Klimakammer im 16 Stunden hell/8 Stunden dunkel-Rhythmus bei 50% Luftfeuchte auf Fremdgenexpression bzw. veränderte Metabolitgehalte und phänotypische Wachstumsmerkmale untersucht. Veränderte Nukleotidgehalte können z.B. nach der Methode von Stitt et al., FEBS Letters, 145(1982), 217-222 bestimmt werden.

### Beispiel 5

### Northernblot-Analyse der transgenen Pflanzen

Die mit dem BinRBP3b-Antisense-Konstrukt (siehe Abbildung 5) transformierten Pflanzen, wurden mittels Northern-Blot-Analysen mit einer strangspezifischen Sonde zur Detektion der ATCase mRNA analysiert. Zur Herstellung der strangspezifischen Sonde wurde pRBP3b mit BamHI gespalten. Die Markierungsreaktion wurde wird mit Hilfe der T7 RNApolymerase durchgeführt (DIG RNA Labeling Kit, Roche Diagnostics). Dabei zeigte sich, daß die Menge der ATCase mRNA in den Linien 8,17, 19, 44, 51, 53 und 60 in unterschiedlichem Maße verringert war.

### Beispiel 6

### Überexpression eines ATCase Fragmentes aus Kartoffel in E.coli und Erzeugung von Antikörpern

Zur Überexpression in E.coli wurde durch Spaltung mit BamHI und HindIII ein 1272 bp Fragment der RBP3b cDNA erhalten und in den ebenso gespaltenen Vektor pQE-9 (Qiagen) ligiert. Das erhaltene Konstrukt QERBP3b codiert für das gesamte ATCase Polypeptid aus RBP3b, dessen N-Terminus mit einem Hexahistidin-Anker sowie 52 weiteren Aminosäuren, die durch DNA-Linkersequenzen und 5'-untranslatierte Sequenzen codiert werden, fusioniert ist. QERBP3b wurde in E. coli XL1-blue (Stratagene) transformiert. IPTG-induzierte Tageskulturen wurden durch Zentrifugation geerntet. Die Zellpellets wurden nach Herstellerangaben unter denaturierenden Bedingungen aufgeschlossen und das ATCase Protein über Nickel-Affinitätschromatographie aufgereinigt ("Qia-Express-Kit", Qiagen). Auf diese Weise konnte die ATCase auf > 95 % gereinigt werden. Das Protein wurde nach üblichen Protokollen zur Erzeugung von Antiseren in Kaninchen verwendet (z.B. als Auftrag bei der Fa. Eurogentec).

### Beispiel 7

### Nachweis der ATCase aus Kartoffel in Blattgeweben

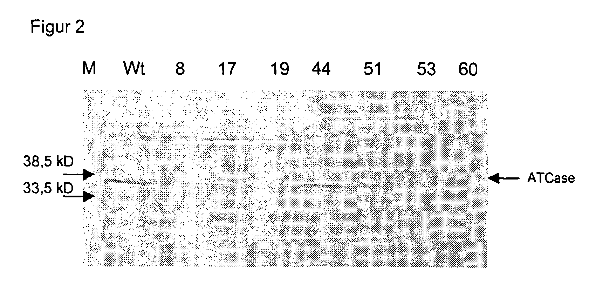

Das ATCase-Antiserum erkennt in Western-Blot Experimenten spezifisch das Polypeptid aus Kartoffel in Form einer Bande bei einem Molekulargewicht von ca. 36 kD. Mithilfe des Antiserums wurden transgene Pflanzen, die ein Antisensekonstrukt der ATCase tragen charakterisiert. Die Linien 8, 17, 19, 44, 51, 53 und 60 zeigen eine unterschiedlich starke Verringerung der ATCase Proteinmenge im Vergleich zum Wildtyp. Abbildung 2 zeigt die Western-Blot Analyse der ATCase Proteinmenge in source-Blättern.

### Beispiel 8

### Phänotypische Analyse der transgenen Pflanzen

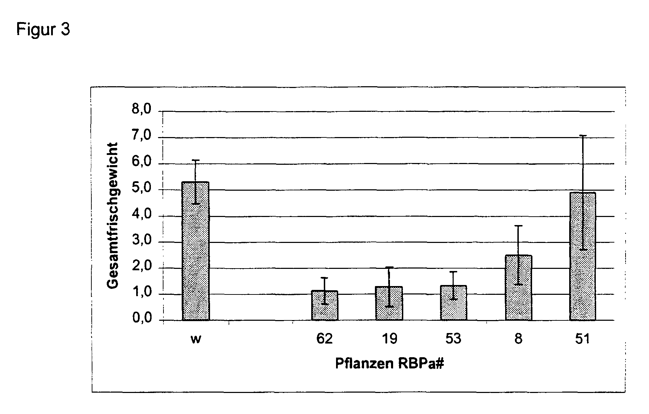

Pflanzen, die ein Antisensekonstrukt der ATCase tragen und eine Verringerung der ATCase-Proteinmenge aufwiesen, zeigen nach 5 Wochen im Gewächshaus in unterschiedlichem Maße eine Wachstumsretardierung, die zu einem verringerten Frischgewicht führt, siehe Tabelle 1 und Abbildung 3. Diese Korellation zwischen Wachstumsretardierung und Reduktion der ATCase Proteinmenge weist die ATC-ase erstmals eindeutig als Herbizidtarget aus.

**Tabelle 1:**

| Analyse der Gesamtfrischgewichte - Mittelwert von 5 bis 8 Replikaten | | |
|---|---|---|
| Pflanze | MW FG [g] | Standardabw. |
| Wildtyp w | 5,29 | 0,83 |
| RBPa 62 | 1,12 | 0,51 |
| RBPa 19 | 1,28 | 0,77 |
| RBPa 53 | 1,32 | 0,53 |
| RBPa 8 | 2,50 | 1,14 |
| RBPa 51 | 4,90 | 2,18 |

### Beispiel 9

### Messung der ATCase-Aktivität

Die benötigte ATCase-Aktivität kann aus Pflanzengeweben präpariert werden, indem zum Beispiel Blattmaterial unter Kühlung mit flüssigem Stickstoff homogenisiert und in Extraktionspuffer (beispielsweise: 2 ml / 100 mg Frischgewicht; 100 mM HEPES, pH 8.0, 2 mM EDTA, 1 mM DTE, 0,1 mM PEFA-Block SC, 10 % Glycerin) gelöst wird.

Alternativ kann eine pflanzliche ATCase (vorzugsweise ATCase aus Kartoffel) in einem geeigneten prokaryontischen (z.B. *E.coli*) oder eukaryontischen (z.B. Hefen, Baculovirus-infizierten Insektenzellen) Expressionssystem hergestellt werden. Hierzu müssen Expressionsvektoren erzeugt werden, die die ATCase cDNA enthalten. Eine modifizierte Länge des N-Terminus der codierten ATCase kann erforderlich sein, um ggf. negative Effekte des N-terminal vorhandenen plastidären Transitpeptides auf die Aktivität des exprimierten Proteins auszuschließen. Geeignete Expressionsvektoren für die Expression in *E.coli* schließen beispielsweise die Typen pQE- (Qiagen), pET-(Novagen), pTrc99a, pKK233-3 (Pharmacia), pBluescript II (Stratagene) ein. Zur Erzeugung rekombinanter Baculoviren für die Expression in Insektenzellen kann beispielsweise das Bac-toBac System (Gibco BRL) oder das MaxBac System (Invitrogen) eingesetzt werden. Zur Expression in Hefe können Vektoren der Serie pYES (Invitrogen) geeignet sein.

Nach Aufschluß der Zellen in geeigneten Puffern und ggf. weiteren Aufreinigungsschritten z.B. über Ammoniumsulfatpräzipitation oder chromatographische Methoden, kann die ATCase-Aktivität zum Beispiel des gebildeten Carbamylaspartats analog zu beschriebenen Verfahren (Prescott & Jones, Anal. Biochem 32 (1969), 406-419; Else & Herve, Anal. Biochem 186 (1990), 219-221) gemessen werden. Ein Aliquot der Enzymreaktion wird dazu mit einer schwefelsauren Farblösung, enthaltend Antipyrine und 2,3-Butanedione Monoxime im dunkeln für mindestens vier Stunden bei 40-60 °C inkubiert. Der gebildete Farbstoff kann photometrisch bei 468nm quantifitziert werden.

Alternativ kann die Umsetzung von [¹⁴C]Carbamylphosphat in das säurestabile [¹⁴C]Carbamylaspartat verfolgt werden (Lovatt und Cheng, Plant Physiology 75(1984), 511-515). Diese Methode zeichnet sich insbesondere durch ihre Sensitivität aus.

Besonders geeignet für eine Messung der ATCase Aktivität im großen Durchsatz ist die von Wedler et al., Analytical Biochemistry 218(1994), 449-453 beschriebene Methode zum Nachweis des entstehenden anorganischen Phosphats durch Umsetzung mit Purinnukleosid Phosphorylase.

Durch Übertragung der Nachweismethode in den Mikrotiterplattenmaßstab, kann im Hochdurchsatz nach Inhibitoren der ATCase-Aktivität gesucht werden, die eine herbizide Wirkung aufweisen.

### Legende zu den Figuren

- Figur 1:: (A) Schematische Darstellung des Expressionsvektors pBlueskript SK enthaltend eine cDNA kodierend für eine ATCase aus Kartoffel (RBP3b) sowie Promotoren der Bakteriophagen T3 bzw. T7. Singuläre Erkennungsstellen für Restriktionsendonukleasen sind eingezeichnet.
(B) Schematische Darstellung des Pflanzentransformationsvektors BinRBP3b enthaltend eine cDNA kodierend für eine ATCase aus Kartoffel (RBP3b) in antisense-Orientierung in dem Vektor pBinAR sowie den 35S-Promotor des Cauliflower Mosaikvirus (CaMV) und den Terminator der Octopinsynthetase (OCS) aus Agrobacterium tumefaciens. Singuläre Erkennungsstellen für Restriktionsendonukleasen sind eingezeichnet.
- Figur 2:: Western-Blot-Analyse zum Nachweis der ATCase aus Kartoffel in Blattgewebe. Dargestellt ist der Einsatz eines Molekulargewichtsmarkers (M) sowie Extrakte der transgenen Pflanzenlinien 8, 17, 19, 44, 51, 53 und 60 im Vergleich zum Wildtyp (WT) zur Hybridisierung mit ATCase-Antiserum.
- Figur 3:: Korrelation zwischen der Wachstumsretardierung (in Gesamtfrischgewicht in Gramm) und der Reduktion der ATCase Proteinmenge der Pflanzenlinien 62, 19, 53, 8 und 51 im Vergleich zum Wildtyp (W).

## Patentansprüche

1. Verwendung einer DNA-Sequenz SEQ ID NO:1, SEQ ID NO:3 oder SEQ ID NO:5, enthaltend die Kodierregion einer pflanzlichen Aspartat-Carbamyltransferase, zur Herstellung eines Testsystems zur Identifizierung von Inhibitoren der pflanzlichen Aspartat-Carbamyltransferase mit herbizider Wirkung.

2. Verwendung einer DNA-Sequenz gemäß Anspruch 1, die für ein Protein kodiert, das die biologische Aktivität einer pflanzlichen Aspartat-Carbamyltransferase besitzt.

3. Verwendung einer DNA-Sequenz gemäß Anspruch 1 oder 2 zur Einführung in pro- oder eukaryontische Zellen, wobei diese Sequenz mit Steuerelementen, die die Transkription und Translation in den Zellen gewährleisten, verknüpft ist und zur Expression einer translatierbaren mRNA, die die Synthese einer pflanzlichen Aspartat-Carbamyltransferase bewirkt, führt.

4. Verwendung eines Proteins mit Aspartat-Carbamyltransferase Aktivität zur Herstellung eines Testsystems zur Identifizierung von Substanzen mit herbizider Wirkung, die die pflanzliche Aspartat-Carbamyltransferase inhibieren mit einer Aminosäuresequenzidentität zu der Aspartat-Carbamyltransferase mit den SEQ ID NO:2 bzw. SEQ ID NO:4, bzw. SEQ ID NO:6 von 80 - 100 %.

5. Verwendung eines Proteins mit Aspartat-Carbamyltransferase Aktivität gemäß Anspruch 4, **dadurch gekennzeichnet, daß** das Protein die in SEQ ID NO:2, SEQ ID NO:4 oder SEQ ID NO:6 dargestellte Aminosäuresequenz, enthält.

6. Verwendung pflanzlicher Proteine mit Aspartat-Carbamyltransferase Aktivität mit einer Aminosäuresequenzidentität zu der Aspartat-Carbamyltransferase aus Kartoffel oder Physcomitrella patens mit den SEQ ID NO:2 bzw. SEQ ID NO:4, bzw. SEQ ID NO:6 von 80 - 100 % zur Herstellung eines Testsystems zum Auffinden von Verbindungen mit herbizider Wirkung.

7. Verfahren zum Auffinden von Substanzen mit herbizider Wirkung, die die Aktivität der pflanzlichen Aspartat-Carbamyltransferase inhibieren, **dadurch gekennzeichnet, daß** in einem ersten Schritt unter Verwendung einer DNA-Sequenz nach Anspruch 1 , 2 oder 3 Aspartat-Carbamyltransferase hergestellt wird und in einem zweiten Schritt die Aktivität der pflanzlichen Aspartat-Carbamyltransferase in Anwesenheit einer Testsubstanz gemessen wird.

8. Verfahren, nach Anspruch 7 **dadurch gekennzeichnet, daß** die Messung der pflanzlichen Aspartat-Carbamyltransferase in einem High-Throughput-Screening (HTS) ausgeführt wird.

9. Verfahren zur Identifizierung von Substanzen mit herbizider Wirkung, die die Aspartat-Carbamyltransferase Aktivität in Pflanzen hemmen, bestehend aus
a) der Herstellung von transgenen Pflanzen, Pflanzengeweben, oder Pflanzenzellen, die eine zusätzliche DNA-Sequenz codierend für ein Enzym mit Aspartat-Carbamyltransferase Aktivität enthalten und in der Lage sind eine enzymatisch aktive Aspartat-Carbamyltransferase überzuexprimieren;
b) das Aufbringen einer Substanz auf transgene Pflanzen, Pflanzenzellen, Pflanzengewebe oder Pflanzenteile sowie auf nicht-transformierten Pflanzen, Pflanzenzellen, Pflanzengewebe oder Pflanzenteile;
c) das Bestimmen des Wachstums oder der Überlebensfähigkeit der transgenen und der nicht-transformierten Pflanzen, Pflanzenzellen, Pflanzengewebe oder Pflanzenteile nach der Aufbringung der chemischen Substanz; und
d) dem Vergleich des Wachstums oder der Überlebensfähigkeit der transgenen und der nicht-transformierten Pflanzen, Pflanzenzellen, Pflanzengewebe oder Pflanzenteile nach der Aufbringung der chemischen Substanz;
wobei die Unterdrückung des Wachstums oder der Überlebensfähigkeit der nicht-transformierten Pflanzen, Pflanzenzellen, Pflanzengewebe oder Pflanzenteile ohne jedoch das Wachstum oder die Überlebensfähigkeit der transgenen Pflanzen, Pflanzenzellen, Pflanzengewebe oder Pflanzenteile zu unterdrücken, belegt, daß die Substanz aus b) herbizide Aktivität zeigt und die Aspartat-Carbamyltransferase Enzymaktivität in Pflanzen inhibiert, und wobei das Enzym Aspartat-Carbamyltransferase eine Aminosäuresequenzidentität zu der Aspartat-Carbamyltransferase mit den SEQ ID NO:2 bzw. SEQ ID NO:4, bzw. SEQ ID NO:6 von 80 - 100 % zeigt.

10. Testsystem **gekennzeichnet durch** die mit Hilfe einer Expressionskassette exprimierte DNA-Sequenz mit 80 - 100 % Identität zu SEQ ID NO:1, SEQ ID NO:3 oder SEQ ID NO:5 kodierend für das Enzym Aspartat-Carbamyltransferase zur Identifizierung von Inhibitoren pflanzlicher Aspartat-Carbamyltransferase mit herbizider Wirkung.

11. Testsystem gemäß Anspruch 10 zur Identifizierung von Inhibitoren pflanzlicher Aspartat-Carbamyltransferase, **dadurch gekennzeichnet, daß** das von einer DNA-Sequenz mit einer Identität von 80 - 100 % zu SEQ ID NO:1, SEQ ID NO: 3 oder SEQ ID NO:5 exprimierte Enzym mit einem zu untersuchenden Testsubstrat inkubiert und nach einer geeigneten Reaktionszeit die enzymatische Aktivität des Enzyms im Vergleich zur Aktivität des nicht gehemmten Enzyms ermittelt wird.

## Claims

1. The use of a DNA sequence SEQ ID NO:1, SEQ ID NO:3 or SEQ ID NO:5 comprising the coding region of a plant aspartate carbamyltransferase for generating an assay system for identifying herbicidally active plant aspartate carbamyltransferase inhibitors.

2. The use of a DNA sequence according to claim 1 which encodes a protein which has the bioactivity of a plant aspartate carbamyltransferase.

3. The use of a DNA sequence according to claim 1 or 2 for introduction into pro- or eukaryotic cells, this sequence being linked to control elements which ensure transcription and translation in the cells and leading to the expression of a translatable mRNA which causes the synthesis of a plant aspartate carbamyltransferase.

4. The use of a protein with aspartate carbamyltransferase activity for generating an assay system for identifying herbicidally active substances which inhibit plant aspartate carbamyltransferase having an amino acid sequence identity to the aspartate carbamyltransferase with the SEQ ID NO:2 or SEQ ID NO:4, or SEQ ID NO:6 of 80 - 100%.

5. The use of a protein with aspartate carbamyltransferase activity according to claim 4, **characterized in that** the protein comprises the amino acid sequence shown in SEQ ID NO:2, SEQ ID NO:4 or SEQ ID NO:6.

6. The use of plant proteins with aspartate carbamyltransferase activity having an amino acid sequence identity to the aspartate carbamyltransferase from potato or Physcomitrella patens with the SEQ ID NO:2 or SEQ ID NO:4, or SEQ ID NO:6 of 80 - 100% for generating an assay system for finding herbicidally active compounds.

7. A method for finding herbicidally active substances which inhibit the activity of plant aspartate carbamyltransferase, **characterized in that**, in a first step, aspartate carbamyltransferase is generated using a DNA sequence as set forth in claim 1, 2 or 3, and, in a second step, the activity of the plant aspartate carbamyltransferase is measured in the presence of a test substance.

8. The method according to claim 7, **characterized in that** the plant aspartate carbamyltransferase is measured in a high-throughput screening (HTS).

9. A method for identifying herbicidally active substances which inhibit the aspartate carbamyltransferase activity in plants, which method comprises
a) generating transgenic plants, plant tissues or plant cells which comprise an additional DNA sequence encoding an enzyme with aspartate carbamyltransferase activity and which are capable of overexpressing an enzymatically active aspartate carbamyltransferase;
**b)** applying a substance to transgenic plants, plant cells, plant tissue or plant parts and to untransformed plants, plant cells, plant tissue or plant parts;
**c)** determining the growth or the survival capacity of the transgenic and the untransformed plants, plant cells, plant tissue or plant parts after applying the chemical substance; and
**d)** comparing the growth or the survival capacity of the transgenic and the untransformed plants, plant cells, plant tissue or plant parts after applying the chemical substance;
where suppression of the growth or the survival capacity of the untransformed plants, plant cells, plant tissue or plant parts without, however, suppressing the growth or the survival capacity of the transgenic plants, plant cells, plant tissue or plant parts, confirms that the substance of b) is herbicidally active and inhibits the aspartate carbamyltransferase enzyme activity in plants, and where the enzyme aspartate carbamyltransferase exhibits an amino acid sequence identity to the aspartate carbamyltransferase with the SEQ ID NO:2 or SEQ ID NO:4, or SEQ ID NO:6 of 80 - 100%

10. An assay system with the DNA sequence expressed with the aid of an expression cassette and exhibiting 80 - 100% identity to SEQ ID NO:1, SEQ ID NO:3 or SEQ ID NO:5 coding for the enzyme aspartate carbamyltransferase for identifying herbicidally active plant aspartate carbamyltransferase inhibitors.

11. The assay system according to claim 10 for identifying plant aspartate carbamyltransferase inhibitors, which comprises incubating the enzyme expressed by a DNA sequence having an identity of 80 - 100% to SEQ ID NO:1, SEQ ID NO:3 or SEQ ID NO:5 with an assay substrate to be studied and, after a suitable reaction time, determining the enzymatic activity of the enzyme compared with the activity of the uninhibited enzyme.

## Revendications

1. Utilisation d'une séquence d'ADN SEQ ID N°1, SEQ ID N°3 ou SEQ ID N°5, contenant la région codante d'une aspartate-carbamyltransférase végétale, pour préparer un système d'essai destiné à l'identification d'inhibiteurs de l'aspartate-carbamyltransférase végétale ayant une activité herbicide.

2. Utilisation d'une séquence d'ADN selon la revendication 1, qui code pour une protéine qui possède l'activité biologique d'une aspartate-carbamyltransférase végétale.

3. Utilisation d'une séquence d'ADN selon la revendication 1 ou 2 pour introduction dans des cellules procaryotes ou eucaryotes, cette séquence étant liée à des éléments de pilotage qui garantissent la transcription et la traduction dans les cellules, et conduisant à l'expression d'un ARNm traductible, qui provoque la synthèse d'une aspartate-carbamyltransférase végétale.

4. Utilisation d'une protéine ayant une activité d'aspartate-carbamyltransférase pour préparer un système d'essai destiné à l'identification de substances ayant une activité herbicide, qui inhibent l'aspartate-carbamyltransférase végétale avec une identité de séquence d'acides aminés avec aspartate-carbamyltransférase des séquences SEQ ID N°2 ou SEQ ID N°4 ou SEQ ID N°6, de 80 à 100 %.

5. Utilisation d'une protéine ayant une activité d'aspartate-carbamyltransférase selon la revendication 4, **caractérisée en ce que** la protéine contient la séquence d'acides aminés représentée dans SEQ ID N°2, SEQ ID N°4 ou SEQ ID N°6.

6. Utilisation de protéines végétales ayant une activité d'aspartate-carbamyltransférase, présentant une identité de séquence d'acides aminés avec l'aspartate-carbamyltransférase de la pomme de terre ou de *Physcomitrella patens* présentant la séquence SEQ ID N°2 ou SEQ ID N°4 ou SEQ ID N°6, de 80 à 100 %, pour préparer un système d'essai destiné à découvrir des composés à activité herbicide.

7. Procédé pour découvrir des substances ayant une activité herbicide, qui inhibent l'activité de l'aspartate-carbamyltransférase végétale, **caractérisé en ce que**, dans une première étape, et par utilisation d'une séquence d'ADN selon la revendication 1, 2 ou 3, on prépare une aspartate-carbamyltransférase, et, dans une deuxième étape, on mesure l'activité de l'aspartate-carbamyltransférase végétale en présence d'une substance d'essai.

8. Procédé selon la revendication 7, **caractérisé en ce que** la mesure de l'aspartate-carbamyltransférase végétale est effectuée dans le cadre d'un High-Throughput-Screening (HTS, criblage à haut débit).

9. Procédé pour identifier des substances ayant une activité herbicide, qui inhibent dans les plantes l'activité aspartate-carbamyltransférase, consistant en
a) la préparation de plantes, de tissus végétaux ou de cellules végétales transgéniques, qui contiennent une séquence d'ADN supplémentaire codant pour une enzyme ayant une activité d'aspartate-carbamyltransférase, et qui sont à même de surexprimer une aspartate-carbamoyltransférase à activité enzymatique ;
b) l'application d'une substance sur des plantes, des cellules végétales, des tissus végétaux ou des parties de plantes transgéniques, ainsi que sur des plantes, des cellules végétales, des tissus végétaux ou des parties de plantes non transformés ;
c) la détermination de la croissance ou de la capacité de survie des plantes, des cellules végétales, des tissus végétaux ou des parties de plantes transgéniques et non transformés, après application de la substance chimique ; et
d) la comparaison de la croissance ou de la capacité de survie des plantes, des cellules végétales, des tissus végétaux ou des parties de plantes transgéniques et non transformés, après application de la substance chimique ;
où la suppression de la croissance ou de la capacité de survie des plantes, des cellules végétales, des tissus végétaux ou des parties de plantes non transformés, sans cependant trop supprimer la croissance ou la capacité de survie des plantes, des cellules végétales, des tissus végétaux ou des parties de plantes transgéniques, apporte la preuve que la substance de b) présente une activité herbicide et inhibe dans les plantes l'activité enzymatique d'aspartate-carbamyltransférase, l'enzyme aspartate-carbamyltransférase présentant une identité de séquence d'acides aminés, avec l'aspartate-carbamyltransférase ayant la séquence SEQ ID N°2 ou SEQ ID N°4 ou SEQ ID N°6, de 80 à 100 %.

10. Système d'essai, **caractérisé par** une identité de séquence d'ADN exprimée à l'aide d'une cassette d'expression de 80 à 100 % par rapport à SEQ ID N°1, SEQ ID N°3 ou SEQ ID N°5 codant pour l'enzyme aspartate-carbamyltransférase, pour identifier des inhibiteurs de l'aspartate-carbamyltransférase végétale ayant une activité herbicide.

11. Système d'essai selon la revendication 10 pour l'identification d'inhibiteurs de l'aspartate-carbamyltransférase végétale, **caractérisé en ce que** l'enzyme exprimée par une séquence d'ADN ayant une identité de 80 à 100 % avec SEQ ID N°1, SEQ ID N°3 ou SEQ ID N°5, est incubée avec le substrat d'essai à étudier et, après un temps de réaction approprié, on détermine l'activité enzymatique de l'enzyme par comparaison avec l'activité de l'enzyme non inhibée.
